Europäisches Patentamt

European Patent Office

Office européen des brevets

(19)

(11) Veröffentlichungsnummer: **0 520 172 B1**

(12) **EUROPÄISCHE PATENTSCHRIFT**

(45) Veröffentlichungstag der Patentschrift: **20.12.95**

(51) Int. Cl.6: **C05F 9/00**, C12M 1/107

(21) Anmeldenummer: **92107886.1**

(22) Anmeldetag: **11.05.92**

(54) **Aufbereitung von Stoffgemischen, insbesondere von Biomüll, Nassmüll, Restmüll und Gewerbeabfällen, für die anaerobe Vergärung der darin enthaltenen biogen-organischen Bestandteile der Stoffe**

(30) Priorität: **24.06.91 DE 4120808**

(43) Veröffentlichungstag der Anmeldung:
**30.12.92 Patentblatt 92/53**

(45) Bekanntmachung des Hinweises auf die Patenterteilung:
**20.12.95 Patentblatt 95/51**

(84) Benannte Vertragsstaaten:
**AT BE CH DE DK ES FR GB GR IT LI LU MC NL PT SE**

(56) Entgegenhaltungen:
**EP-A- 0 286 100
EP-A- 0 291 425
DE-A- 3 225 026**

(73) Patentinhaber: **REA GESELLSCHAFT FÜR RECYCLING VON ENERGIE UND ABFALL MBH
Rottmannstrasse 18
D-80333 München (DE)**

(72) Erfinder: **Wiljan, Harry
Hans Sachs-Strasse 8
W-8000 München 5 (DE)**
Erfinder: **Niefnecker, Ulrich**
**Leutstettener Strasse 20
W-8000 München 71 (DE)**
Erfinder: **Muck, Ottokarl
Oranienstrasse 45
W-1000 Berlin 61 (DE)**
Erfinder: **Kübler, Hans
Gabelsberger Strasse 85
W-8000 München 2 (DE)**
Erfinder: **Schnell, Roland
Möckerstrasse 71
W-1000 Berlin 61 (DE)**
Erfinder: **Carra, Roland
Reischlweg 16
W-8000 München 45 (DE)**
Erfinder: **Wild, Matthias
Kaiserstrasse 46/3
W-8000 München 40 (DE)**

(74) Vertreter: **Reinhard - Skuhra - Weise & Partner
Postfach 44 01 51
D-80750 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren und eine Vorrichtung zur Aufbereitung von Stoffgemischen für die anaerobe Vergärung der darin enthaltenen biogen-organischen Stoffe, die für eine anaerobe Vergärung geeignet sind, insbesondere von Biomüll, Naßmüll, Restmüll und Gewerbeabfällen.

Für die Aufbereitung und Sortierung von Abfällen, sowohl für Haushalts-, als auch für Gewerbeabfälle, werden bisher fast ausschließlich Sortiereinrichtungen verwendet, die eine Trennung der verschiedenen Bestandteile ohne wesentliche Veränderung ihrer Feuchtigkeit durch unterschiedlich angeordnete Kombinationen von Zerkleinerungseinrichtungen, Sieben, Windsichtern und Einrichtungen zur manuellen Sortierung anstreben. Ein Beispiel hierfür ist in der DE-PS 30 37 714 offenbart.

Gelegentlich ist für die Aufbereitung von Abfällen auch schon die Ausnutzung der Trennwirkung von Wasser vorgeschlagen worden, die sowohl als eigenständiges Verfahren als auch als Teilverfahren im Rahmen einer Sortieranlage der oben beschriebenen Bauart durchgeführt werden kann.

So beschreibt die DE-OS 33 25 504 ein Verfahren zur Aufbereitung mit Teilrecycling von Hausmüll, Sperrmüll, im wesentlichen aus Verpackungsabfall bestehendem Gewerbemüll und kommunalem Klärschlamm sowie Einrichtungen zur Ausübung des Verfahrens, bei dem die Abfälle erst zerkleinert und in drei Siebfraktionen geschieden werden. Für die Mittelfraktion wird eine weitere Fraktionierung durch Einbringen in eine mit Wasser gefüllte Wanne vorgeschlagen, in der die anorganischen Schwerstoffe zu Boden sinken sollen, während die Leichtstoffe aufschwimmen, so daß sie abgeschöpft und einer Kompostierung oder anaeroben Vergärung zugeführt werden können.

Eine ähnliche Anordnung beschreibt die DE-OS 38 36 379, betreffend ein Verfahren zum Aufbereiten von Naßmüll zum Vergären von dessen organischen Komponenten zusammen mit Klärschlamm und Vorrichtung zum mechanischen Trennen von organischen und nichtorganischen Müllanteilen, bei dem die abgesiebte Mittelfraktion durch eine Vorrichtung aufbereitet wird, in der mit Hilfe von Stachelwalzen eine Trennung der organischen von den anorganischen Anteilen erreicht werden soll.

In der DE-OS 35 00 132 wird ein Verfahren zur Gewinnung und Trennung organischer Bestandteile aus dem Müll beschrieben, bei dem die leichten Bestandteile ohne vorherige Zerkleinerung durch Ausblasen mit Luft aus dem Abfallgemisch abgetrennt und in ein Wasserbecken transportiert werden sollen. Dort sollen sich die schweren Stoffe absetzen, während die aufschwimmenden Stoffe durch eine leichte Strömung zu einer Austragvorrichtung gefördert werden sollen.

Eine Trennung von Abfällen in ruhendem oder nur leicht bewegten Wasser ergibt jedoch nur einen sehr unvollkommenen Effekt. Die Sinkgeschwindigkeit der Fremdstoffe, insbesondere der Feinanteile ist gering, und die Abtrennung von Kunststoffen, deren spezifisches Gewicht sich nur wenig vom spezifischen Gewicht der organischen Stoffe unterscheidet, ist nicht möglich. In der DE-OS 35 00 132 ist deshalb vorgesehen, die aufschwimmenden Abfälle zu kochen, wodurch die Struktur der organischen Stoffe soweit zerstört wird, daß sie durch ein Feinsieb gepreßt und von den thermisch widerstandsfähigeren Komponenten, beispielsweise den Kunststoffen getrennt werden können. Nachteilig sind hierbei allerdings der hohe technische Aufwand und der hohe Energiebedarf, der zur Durchführung dieses Verfahrens erforderlich ist.

Weiterhin wird in nachteiliger Weise für die Trennung im ruhenden oder leicht bewegten Wasser eine im Verhältnis zu den Feststoffen sehr große Wassermenge benötigt, wobei in den genannten Verfahren keine Angaben darüber gemacht werden, wie das mit gelösten und fein suspendierten organischen und anorganischen Stoffen belastete Wasser weiterverwertet oder gereinigt werden soll.

Bei Abfällen, die mit einem biotechnischen Verfahren weiterbehandelt werden sollen, ist es insbesondere dann, wenn eine Erzeugung von Biogas angestrebt wird, sinnvoll, zur Erhöhung der Reaktionsgeschwindigkeit die organischen Stoffe vor, nach oder während der Abtrennung zu zerkleinern. In der DE-OS 38 36 379 wird für die Zerkleinerung nach der Abtrennung der organischen Anteile eine Kugelmühle verwendet. Die Verwendung einer Kugelmühle bewirkt, daß die organischen Stoffe, wie in der DE-OS 38 36 379 beschrieben, zu einer breiartigen Konsistenz vermust werden. Die so zerkleinerten organischen Stoffe können dann nur noch in konventionellen Biogas-Anlagen nach dem Prinzip des einphasigen Faulraums weiterverwertet werden.

Die mit dem einphasigen, vollständig durchmischten Faulraum verbundenen Probleme und Einschränkungen im Betrieb sind in der Fachliteratur ausführlich dargestellt worden. Es ist bekannt, daß wesentlich leistungsfähigere Biogas-Reaktorsysteme geschaffen werden können, wenn die Auflösung (Hydrolyse) der Feststoffe von der eigentlichen Methanbildung getrennt durchgeführt wird, und dieser nur gelöste organische Stoffe zugeführt werden. Für die Umsetzung gelöster organischer Stoffe zu Biogas stehen bereits leistungsfähige Reaktorsysteme zur Verfügung.

Die zweistufige Vergärung von Feststoffen kann aber nur dann erfolgreich durchgeführt werden, wenn die organischen Stoffe mit hohem Wirkungsgrad von den im Abfallgemisch vorhandenen Fremdstoffen abgetrennt und unter Erhalt ihrer Faserstruktur zerkleinert werden. Mit den bekannten und bisher in der Abfallaufbereitung eingesetzten Zerkleinerungseinrichtungen, insbesondere mit der erwähnten Kugelmühle, ist das nicht möglich. Neben dem bei dieser Form der Zerkleinerung unvermeidlichen "Zermusen" der organischen Abfälle, was eine Abtrennung der ungelösten organischen Feststoffe erschwert, ist damit in nachteiliger Weise immer auch eine starke Zerkleinerung der nichtorganischen Fremdstoffe verbunden, die zu einem unerwünschten Eintrag von Schwermetallen führt.

Eine Abtrennung der Fremdstoffe (Glas, Kunststoffe in Form von Folien und Behältern, Metalle, Steine, Sand) vor der Zerkleinerung ist mit den oben erwähnten Sortiereinrichtungen nur in beschränktem Umfang möglich, da sie mit den feuchten organischen Stoffen innig vermengt und verklebt sind. Für die Aufbereitung von Abfällen mit dem Ziel einer biotechnischen Verwertung ihres organischen Anteils sind daher diese Sortier- und Zerkleinerungseinrichtungen und die aus ihnen gebildeten Aufbereitungsverfahren nicht geeignet.

Für eine Naßaufbereitung ohne Vorsortierung oder Vorzerkleinerung von Abfallstoffen kann ein Stofflöser eingesetzt werden, wie es beispielsweise in der DE-PS 20 47 006 beschrieben wird, Dem Stofflöser werden dabei kontinuierlich gemischte Abfälle zugeführt, die gleichzeitig zerfasert und zerkleinert und kontinuierlich als Suspension durch ein Lochsieb abgezogen werden. Die Zerfaserung betrifft die biogen-organischen Abfallstoffe, vor allem die Papier- und Pappeanteile, Küchen- und Gartenabfälle, während alle anderen Stoffe durch die besondere Ausgestaltung des Laufrades zerbrochen oder zerschnitten werden sollen. Glas soll zerschlagen und Aluminium soll zu Knäueln verdichtet werden, die durch eine relativ große Sieblochung zusammen mit der Fasersuspension abgepumpt und in weiteren Prozeßstufen abgetrennt werden. Die dabei angewandte Methode der Abtrennung der anorganischen und metallischen Anteile von den organischen Stoffen durch Hydrozyklone bedingt, daß der Stofflöser bei einer niederen Feststoffkonzentration betrieben werden muß. Die Fremdstoffe, die weder zerfasert noch zerkleinert werden können, gelangen durch eine Öffnung am Boden in ein spezielles Trenn- und Reinigungssystem, in dem die Fasern abgetrennt und wieder in den Stofflöser zurückgeführt werden.

Die hier angewandte Zerkleinerung der anorganischen Fremdstoffe wirkt sich äußerst nachteilig auf die Qualität der organischen Faserstoffe aus, da durch das Zerkleinem fein verteilte Schwermetalle freigesetzt werden, die sich im Endprodukt wiederfinden und seine landwirtschaftliche Verwendung als Bodenverbesserungsmittel erschweren oder verhindern. Als nachteilig ist insbesondere das Zerschlagen des Glases anzusehen, weil dadurch scharfkantiger Glasbruch entsteht, der zu Abrasion an Pumpen und Rohrleitungen führt und der sich in Rohrleitungen und Behältern absetzt und diese verstopfen kann.

Ein besonderes Problem bei der Aufbereitung von Abfällen mit einem Stofflöser stellen die Kunststoffe in Form von großflächigen Folien und Behältern dar. Bei Altpapier kann aufgrund der charakteristischen Eigenschaften der darin enthaltenen Fremdstoffe erreicht werden, daß Schnüre und Drähte einen sogenannten Zopf bilden, der mit Hilfe eine Winde langsam abgezogen wird. Aber bereits bei Altpapier kann die Menge der Fremdstoffe ein Ausmaß erreichen, daß diese Methode nicht mehr befriedigend funktioniert. In der DE-PS 32 25 026 wird deshalb für diese Aufgabe eine Rechenvorrichtung beschrieben, mit der Grob- und Schwerabfall sicher und mit möglichst geringem Bedienungsaufwand kontinuierlich entfernbar sein soll.

Bei Abfällen, insbesondere bei Restmüll und Gewerbeabfällen, muß davon ausgegangen werden, daß die flächigen (Kunststoffolien) und die voluminösen (Behälter, Flaschen) Anteile nicht in der Lage sind, einen Zopf zu bilden. In der DE-OS 24 06 404, betreffend ein Verfahren zur Rückgewinnung wiederaufbereitbarer Bestandteile aus Abfallmaterial, wird aus diesem Grund das Ausspülen der Kunststoffe aus dem Stofflöser mit einem Teilstrom der Suspension und die Trennung in Fasersuspension und Fremdstoffe in einem zusätzlichen, externen Apparat angewandt. Dafür ist ebenfalls eine niedere Feststoffkonzentration der Suspension im Stofflöser die Voraussetzung.

Gemäß dem in der EP-A-0 291 425 beschriebenen Verfahren werden Abfälle vor der Anwendung hydromechanischer Kräfte mit einem Reißwolf mechanisch.

In der EP-PS 0 286 100 ist bereits ein Verfahren zur Aufbereitung und anaeroben Vergärung biogen-organischer Abfälle und eine Vorrichtung zur Durchführung des Verfahrens beschrieben worden. Bei diesem Verfahren werden zunächst die biologisch nicht verwertbaren Fraktionen aus dem Rohabfall abgetrennt, dann wird in einer Vorbehandlungsstufe die biogen-organische Abfallfraktion durch Zusatz von Wasser oder Flüssigabfall aufgeweicht und gleichzeitig einer kombinierten hydraulischen und mechanischen Schereinwirkung zur Auflösung des Abfallmaterials unter wesentlicher Beibehaltung seiner Faserbeschaffenheit ausgesetzt und dadurch eine Rohsuspension mit 3-15 Gew.-% organischer Trockenmasse erzeugt. Danach wird die Rohsuspension in einer sich anschließenden Laugenstufe durch Zusatz von Chemikalien auf alkalische Bedingungen eingestellt, auf 40-60 °C erwärmt und 2 bis 12 Stunden auf einer Temperatur in diesem Bereich gehalten. Daran anschließend wird die der Laugenstufe entnommene Suspension durch eine Fest-Flüssig-Trennstufe in einen die wasserlöslichen biologischen Stoffe enthaltenden Flüssigkeitsstrom und in

3

einen Feststoffstrom mit 20-50 Gew.-% organischer Trockenmasse aufgeteilt, wobei der Flüssigkeitsstrom anschließend einer Methangärung zugeführt und der Feststoffstrom einer anaeroben Feststoffhydrolyse im sauren Bereich und im meso- oder thermophilen Temperaturbereich ausgesetzt und das dabei gewonnene Hydrolysat der Methangärung zugeführt wird. Eine für die Vorbehandlungsstufe geeignete Stofflöseeinrichtung ist auch in der DE-OS 37 11 813 derselben Anmelderin offenbart.

Die dort beschriebene Vorrichtung und die Betriebsweise dieser Vorrichtung zur Abtrennung einer organischen Faserfraktion ist allerdings nur in unvollkommener Weise geeignet, diese für die nachfolgende anaerobe Behandlung aufzubereiten und die nicht-organischen Bestandteile so abzutrennen, daß sie wiederverwertet bzw. deponiert werden können. Dieser Nachteil tritt umso stärker in Erscheinung, je höher der Anteil der Fremdstoffe in dem verwendeten Abfall ist. Es ist nämlich nur in geringem Umfang möglich, die auf oder in der Suspension schwimmenden nicht verwertbaren Bestandteile des Abfalls während des Auflösevorgangs aus der Stofflösevorrichtung abzuschöpfen. Ebenso unvollkommen ist die Abtrennung der Schwerstoffe, vor allem, wenn eine möglichst hohe Feststoffkonzentration in der Suspension angestrebt wird. Überdies sind die abgetrennten Fremdstoffe mit Faserstoffen behaftet, die eine Ablagerung oder Wiederverwertung der Materialien ohne weitere Aufbereitung verhindern.

Die Folge davon ist, daß die Stofflösevorrichtung wegen der Abtrennung der Fremdstoffe länger betrieben werden muß, als es unter dem Gesichtspunkt der Auflösung und Zerfaserung der organischen Stoffe allein nötig wäre. Durch den längeren Betrieb bei hoher Drehzahl erhöht sich aber der Energieverbrauch, und der Verschleiß nimmt zu.

Eine Verbesserung der Trennwirkung könnte durch Verminderung der Feststoffkonzentration in der Suspension erreicht werden. Dies würde aber den technischen Aufwand und den Energiebedarf in den nachgeschalteten Stufen, insbesondere bei der Fest-Flüssig-Trennung und der Feststoff-Hydrolyse, in unerwünschter Weise erhöhen.

Der Erfindung liegt die Aufgabe zugrunde, ein Verfahren und eine Vorrichtung der eingangs genannten Art verfügbar zu machen, bei der die Abtrennung der gelösten organischen Verbindungen und der organischen Faserstoffe von den nicht biotechnisch verwertbaren Fremdstoffen mit hohem Wirkungsgrad in besonders schonender Weise erfolgt. Erfindungsgemäß wird diese Aufgabe durch die im Patentanspruch 1 bzw. die im Patentanspruch 16 gekennzeichneten Merkmale gelöst.

Bevorzugte weitere Ausgestaltungen der Erfindung sind den jeweils nachgeordneten Patentansprüchen zu entnehmen.

Unter dem Ausdruck "biogen-organische Stoffe" sind solche Stoffe zu verstehen, die für eine anaerobe Vergärung geeignet sind. "Nichtbiogen-organische (Frend) stoffe" sind solche Stoffe, die für eine anaerobe Vergärung ungeeignet sind.

In vorteilhafter Weise werden gemäß dem erfindungsgemäßen Verfahren und der erfindungsgemäßen Vorrichtung die nicht biogen-organischen Fremdstoffe mechanisch außerordentlich wenig beschädigt oder zerkleinert, und im gleichen Maße wird die Abtrennung dieser Feststoffe erleichtert, Außerdem wird der Verschleiß durch abrasive Feinanteile, die beim Zerschlagen spröder Materialien, wie z. B. Glas, zwangsläufig entstehen, vermindert.

Durch das in der Vorbehandlungsstufe auftretende Auflösen und Zerfasern der organischen Stoffe, das Abziehen der Fasersuspension, das Abtrennen der nicht vergärbaren Bestandteile des Abfalls in zeitlich nacheinanderfolgender Form in einem speziell hierfür vorgesehenen Auflösebehälter, der integraler Bestandteil einer Vorrichtung zur Erzeugung von Biogas aus der daraus erzeugten Suspension ist, kann in vorteilhafter Weise eine verschleißarme Aufbereitung mit vergleichsweise geringerem Energieverbrauch erreicht werden.

Durch die vollkommene Abtrennung der Fremdstoffe wird ferner die Vergärbarkeit der Suspension in den nachfolgenden Verfahrensstufen verbessert und ein unerwünschter Eintrag von Schwermetallen in die mit dem Verfahren erzeugten kompostähnlichen Reststoffe vermieden.

Weiterhin wird durch die effektive Abtrennung der biogen-organischen Bestandteile aus dem Ausgangsmaterial die Recycling- und Deponiefähigkeit der abgetrennten Fremdstoffe erhöht.

Weitere bevorzugte Ausgestaltungsformen der Erfindung sind wie folgt:
In der Vorbehandlungsstufe wird die Suspension aus gelösten und zerfaserten biogen-organischen Stoffen diskontinuierlich abgezogen, und die verbleibenden Fremdstoffe werden mit Wasser, vorzugsweise mit Prozeßwasser, derart aufgeschwemmt, daß anschließend die Schwerstoffe zentrifugal abtrennbar und aufbzw. in der Fremdstoffsuspension schwimmende Leichtstoffe abschöpfend oder siebend entnehmbar sind.

Weiterhin wird die mechanische Aufbereitung bevorzugt in einem Auflösebehälter mit einem drehbaren Laufrad durchgeführt, wobei für jede Phase des Auflösungs- und Abtrennvorgangs zur schonenden Behandlung der nicht biogen-organischen Fremdstoffe die Drehzahl des Laufrades angepaßt wird.

Weiterhin bevorzugt wird das Laufrad chargenweise in einer ersten Phase während des Auffüllens mit einer niedrigen Drehzahl während der anschließenden Phase des Aufschlämmens mit einer hohen Drehzahl, während einer folgenden Phase des Abziehens der Suspension bis zum Auffüllen mit Wasser, vorzugsweise Prozeßwasser, mit einer ersten Drehzahl und in einer letzten Phase des Auffüllens mit Wasser, vorzugsweise Prozeßwasser, und während der Entnahme der Leichtstoffe mit einer zweiten Zwischendrehzahl, die kleiner ist als die erste Zwischendrehzahl, angetrieben.

Weiterhin bevorzugt werden die abgeschöpften bzw. siebend entnommenen Leichtstoffe einer zusätzlichen Reinigungsbehandlung unter Verwendung von Wasser, vorzugsweise Prozeßwasser, zum Abziehen von noch an den Leichtstoffen anhaftendem Fasermaterial und dessen Rückführung in den Auflösungsbehälter unterworfen.

Weiterhin bevorzugt werden abgeschöpfte oder abgesiebte Leichtstoffe zerkleinert und noch anhaftende Faserreste abgewaschen.

Weiterhin bevorzugt wird die Einwirkung strömungsmechanischer Kräfte in Abhängigkeit von der Feststoffkonzentration gesteuert.

Nachfolgend wird die Erfindung unter Bezugnahme auf die beigefügten Zeichnungen näher erläutert. Es zeigen:

Fig. 1    ein Fließdiagramm zur Veranschaulichung des Ablaufs des erfindungsgemäßen Verfahrens;
Fig. 2    eine schematische Darstellung der Vorrichtung zur Durchführung des Verfahrens mit den zugehörigen Vorrichtungskomponenten und deren Zusammenwirken;
Fig. 3    ein Diagramm für den Betrieb des Auflösebehälters, in dem der Füllstand, die Leistung und die Drehzahl über der Zeit für einzelne Betriebsphasen angegeben sind;
Fig. 4    eine schematische Darstellung des Auflösebehälters mit zugeordneter Recheneinrichtung; und

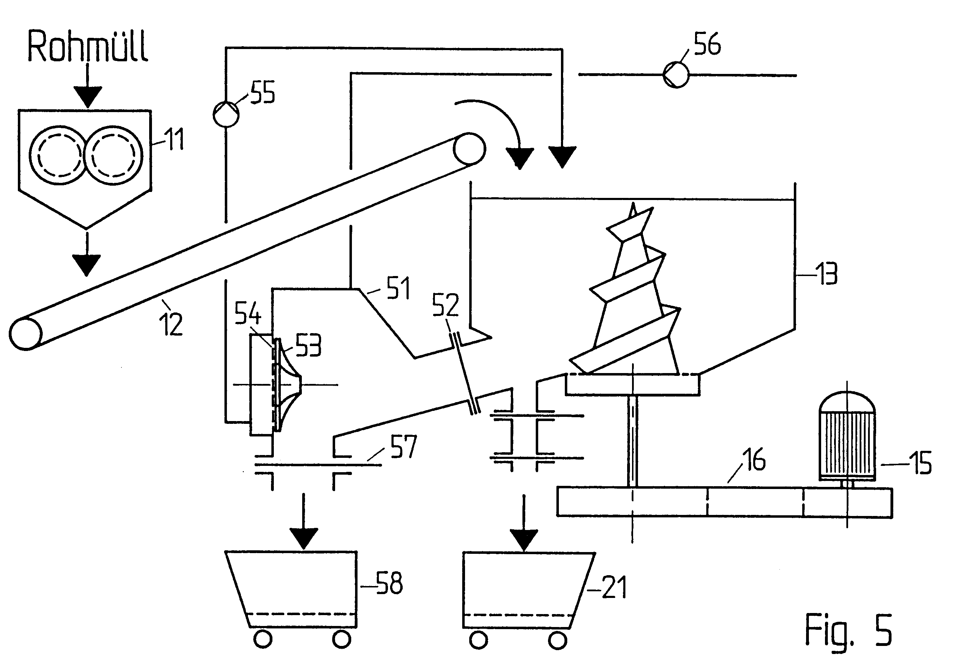

Fig. 5    eine schematische Darstellung des Auflösebehälters mit einer gegenüber der von Figur 4 abgewandelten Einrichtung in Form einer Reinigungskammer statt einer Recheneinrichtung.

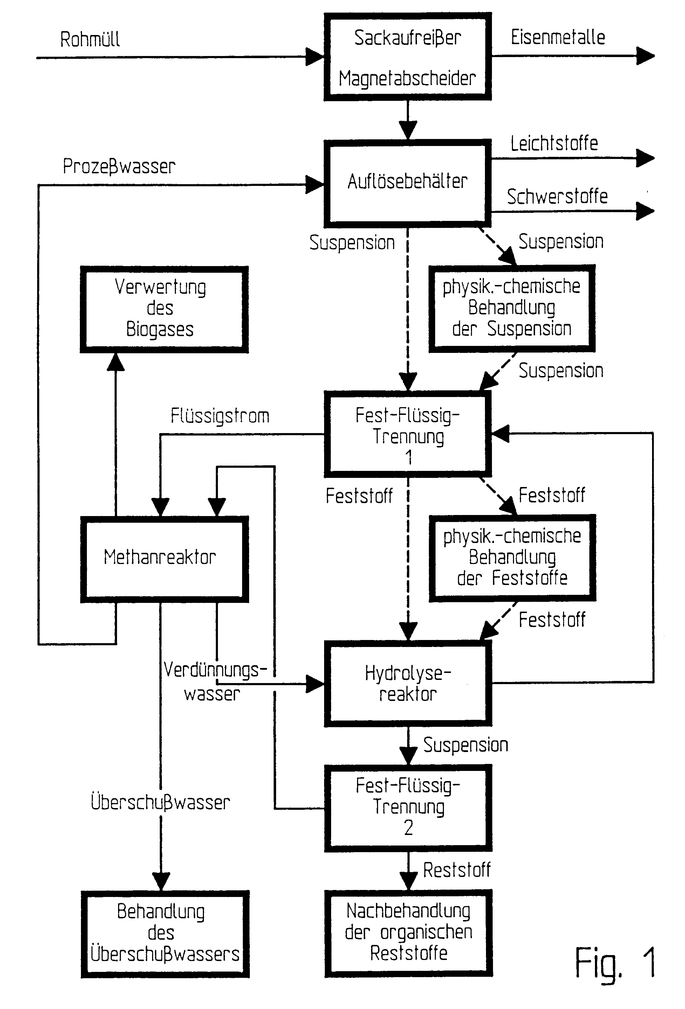

In Fig. 1 ist das erfindungsgemäße Verfahren allgemein in Form eines Fließdiagramms dargestellt. Rohmüll, der aus biogen-organischen und nichtbiogen-organischen Stoffen besteht wird üblicherweise in Säcken angeliefert. In einem Sackaufreißer werden diese Säcke geöffnet, wobei der Rohmüll leicht vorzerkleinert und aufgelockert wird. Danach werden mittels eines Magnetabscheiders Eisenmetalle abgetrennt und dem Recycling zugeführt. Der von Eisenmetallen weitgehend befreite Rohmüll gelangt dann in einen Auflösebehälter, in dem er mit Wasser, insbesondere Prozeßwasser, versetzt und einer kombinierten hydraulischen und mechanischen Scherwirkung ausgesetzt wird, um eine Rohsuspension zu erzeugen, die von Fremdstoffen weitgehenst befreit ist. Nach dem Abpumpen der Suspension und dem erneuten Auffüllen mit Prozeßwasser werden die Schwerstoffe (= Fremdstoffe, die wegen ihres im Vergleich zum Wasser höheren spezifischen Gewichts absinken) abgeführt und die Leichtstoffe (= Fremdstoffe, die wegen ihres annähernd gleichen Gewichts auf bzw. im Prozeßwasser schwimmen) entnommen und ggf. einer weiteren Behandlung unterzogen, um noch anhaftende biogen-organische Substanzen, wie Fasern und dergl., zu beseitigen.

Wahlweise kann die abgepumpte Suspension nun einer physikalisch-chemischen Behandlung unter alkalischen Bedingungen und bei erhöhter Temperatur unterworfen werden, um bei bestimmten Müllsorten die Vergärbarkeit zu erhöhen.

Falls diese Option nicht wahrgenommen wird, erfolgt direkt eine Zuführung der Suspension aus dem Auflösebehälter in eine Fest-Flüssig-Trennstufe, in der eine Auftrennung in einem Flüssigkeitsstrom und einen Feststrom erfolgt. Diese Fest-Flüssig-Trennung (1) wird auch bei der wahlweisen physikalisch-chemischen Behandlung der Suspension als nächste Verfahrensstufe angewandt. Der abgezweigte Flüssigkeitsstrom enthält die wasserlöslichen Stoffe, während der Feststoffstrom 20 - 50 Gew.-% Trockenmasse aufweist.

Der Flüssigkeitsstrom wird einem Methanreaktor zugeführt, der zur Erzeugung von Biogas dient. Das Abwasser des Methanreaktors wird innerhalb der Vorbehandlungsstufe als Prozeßwasser dem Auflösebehälter und als Verdünnungswasser dem Hydrolysereaktor zugeführt. Überschußwasser erfährt eine Behandlung, die eine Abgabe an die Umwelt ermöglicht.

Der aus der ersten Fest-Flüssig-Trennstufe abgehende Feststoffstrom wird dann wahlweise direkt oder nach der physikalisch-chemischen Behandlung der Feststoffe, beispielsweise durch eine Ozonbehandlung, dem Hydrolysereaktor zugeführt und mit Verdünnungswasser aus dem Methanreaktor vermischt. Die Suspension aus der Hydrolyse wird wieder der Fest-Flüssig-Trennung (1) zugeführt, die das Hydrolysat vorn hydrolysierten Feststoffmaterial abtrennt, dieses der Methangärung zuführt und die Feststoffe wieder dem Hydrolysereaktor zuleitet. Dieser Kreislauf über die Fest-Flüssig-Trennstufe (1) erfolgt solange, bis das bearbeitete Hydrolysat keinen praktisch verwertbaren Gehalt an gelösten organischen Stoffen mehr besitzt.

Sobald dieser Zustand erreicht ist, werden die nicht mehr innerhalb des Prozesses verarbeitbaren Feststoffe einer zweiten Fest-Flüssig-Trennung zugeführt, aus der die Feststoffe dann einer Nachbehandlung der organischen Reststoffe in Form eine aeroben Nachrotte zugeleitet werden. Der bei der zweiten Fest-Flüssig-Trennung anfallende Flüssigstrom wird ebenfalls dem Methanreaktor zugeführt.

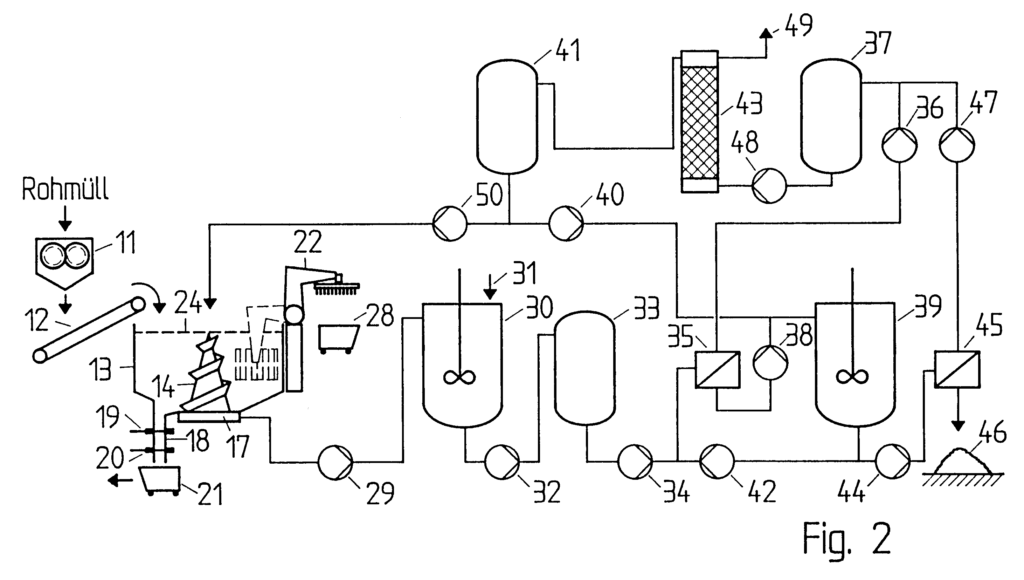

In Fig. 2 sind Vorrichtungsbestandteile schematisiert in ihrer funktionellen Verknüpfung im Rahmen des Verfahrens dargestellt. Die Beschreibung der einzelnen Vorrichtungsbestandteile erfolgt daher dem Verfahrensablauf entsprechend.

Rohmüll, der in der Regel in Müllsäcken verpackt angeliefert wird, passiert zunächst einen Sackaufreißer 11, der die Müllsäcke aufreißt und die Rohmüllbestandteile besser zugänglich macht. Aus dem Sackaufreißer 11 gelangt der Rohmüll dann auf eine Fördereinrichtung, beispielsweise ein Förderband 12, mit dem er chargenweise in einen Auflösebehälter 13 transportiert wird. Bei bestimmten Rohmüll- bzw. Abfallarten kann auch zusätzlich eine nicht dargestellte Absiebung feiner anorganischer Fremdstoffe, eine Aussortierung grober und sperriger Gegenstände oder eine Abtrennung ferromagnetischer Materialien durch einen Magneten vor einer Behandlung in dem Auflösebehälter 13 zweckmäßigerweise vorgesehen sein.

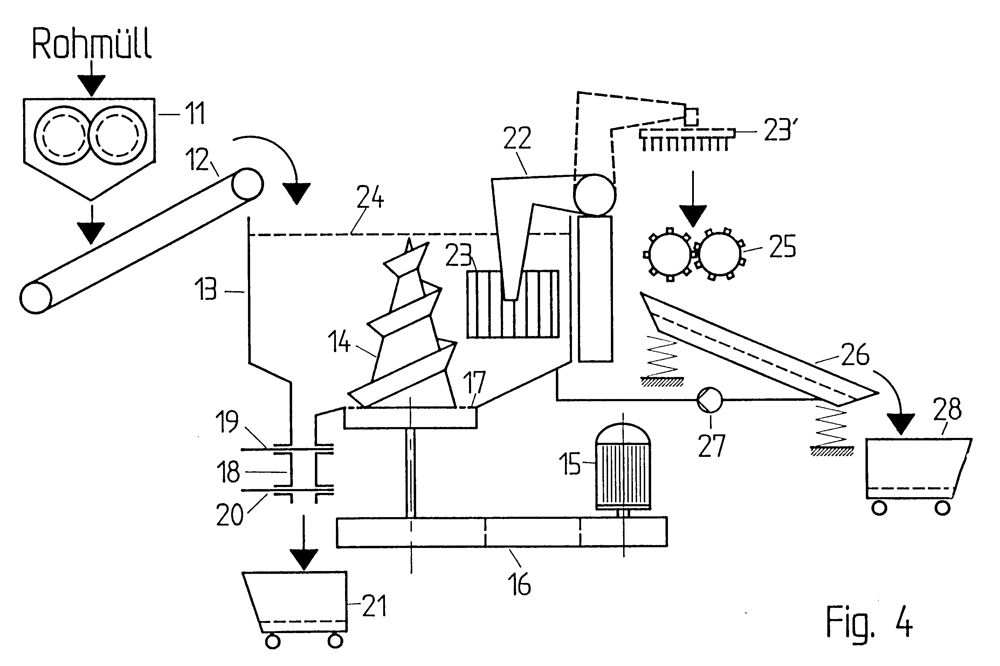

Der Auflösebehälter 13 entspricht in seiner Form im wesentlichen dem aus der Altpapieraufbereitung bekannten Stofflöser bzw. Pulper. Er weist ein zentrales, sich um eine vertikale Achse drehendes Laufrad 14 auf. Das Laufrad 14 wird, wie aus Fig. 4 entnehmbar, mittels eines Motors 15 über einen in jeweilige Riemenscheiben eingreifenden Keilriemen 16 angetrieben. Die Motordrehzahl und damit die Drehzahl des Laufrades ist in jeder Betriebsphase des Auflösebehälters 13 durch eine nicht dargestellte elektronische Steuerung auf einen bestimmten vorgegebenen Wert einstellbar.

Unterhalb des Laufrades 14 befindet sich in der Mitte des Auflösebehälters 13 ein Lochsieb 17 in Form einer Siebplatte. Der Lochdurchmesser des Lochsiebes 17 beträgt etwa 8 bis 12 mm. Am Boden des Auflösebehälters 13 ist weiterhin eine Schwerstoffschleuse 18 angeordnet, die durch eine Schleusenkammer gebildet wird, die von einem oberen Schieber 19 und einem unteren Schieber 20 begrenzt wird. Unterhalb der Schwerstoffschleuse 18 befindet sich ein Behälter 21 zur Sammlung und zum Abtransport der Schwerstoffe.

Das Laufrad 14 ist kegelähnlich ausgebildet, wobei auf dem Kegelmantel ein spiralförmig sich von unten nach oben erstreckendes, von dem Mantel des Laufrads um etwa 45° abgewinkeltes Leitblech vorgesehen ist. Bei Rotation des Laufrades 14 wird im Innern des Auflösebehälters 13 eine torroidale bzw. schleifenförmige Strömung in der Suspenison erzeugt, die am Boden radial nach außen gerichtet ist und eine geringe tangentialen Strömungskomponente aufweist. Die von dem Laufrad 14 initiierte Strömung führt zu dem Auftreten von hydraulischen Scherkräften in der Suspension, welche das Zerfasern aufgeweichter biogen-organischer Stoffe bewirken.

Dem Auflösebehälter 13 ist eine Recheneinrichtung 22 zugeordnet, der eine vertikal stellbare Rechenfläche 23 mit Rechenzinken 23' aufweist, welche zum Festhalten von Leichtstoffen vertikal stellbar und unter den Flüssigkeitsspiegel 24 im Auflösebehälter 13 eintauchbar ist. Die Rechenfläche 23 ist aus der in Fig. 4 mit ausgezogenen Linien dargestellten Eintauchstellung aus dem Auflösebehälter 13 in die gestrichelt dargestellte Position zum Herausbefördern der aufgefangenen Leichtstoffe schwenkbar. Aus diesem ausgefahrenen Zustand werden Leichtstoffe auf einen Shredder 25 zur Aufbereitung abgeworfen, in dem sie derart zerkleinert werden, daß sie auf einem darunter angeordneten Vibrationssortierer 26 entwässert werden können. Das bei dem Vibrationssortierer 26 abtropfende Wasser wird mittels einer Pumpe 27 wieder in den Auflösebehälter 13 zurückgepumpt, vgl. Fig. 4. Die aufbereiteten Leichtstoffe werden dann in einen Behälter 28 abgeworfen und abtransportiert.

Nach der Entnahme der Leichtstoffe steht der Auflösebehälter 13 wieder bereit für die Aufnahme einer neuen Charge des Rohmülls bzw. Abfalls, nachdem gemäß Fig. 2 die Rohsuspension mit Hilfe einer Pumpe 29 durch die Siebplatte 17 abgepumpt und einem Rührreaktor 30 für eine thermisch-chemische Vorbehandlung zugeführt worden ist. Durch diese thermische und chemische Behandlung wird der Anteil der gelösten organischen Stoffe erhöht, die Suspension hygienisiert und ihre Entwässerbarkeit verbessert. Die thermisch-chemische Behandlung kann bei Temperaturen von 50-90° durchgeführt werden. Sie wird vorzugsweise als alkalische Behandlung zwischen 60 und 70°C vorgenommen, wobei durch die Zugabe von Lauge (Bezugszeichen 31), vorzugsweise Natronlauge, auch eine Verseifung der Fette bewirkt wird, die damit in die wasserlösliche Form überführt werden.

Nach Beendigung der thermisch-chemischen Behandlung wird die Suspension mit Hilfe einer Pumpe 32 in einen Suspensionspuffer 33 überführt, aus dem sie kontinuierlich mit Hilfe einer Dosierpumpe 34 einer Fest-Flüssig-Trennung 35 zugeführt wird, von der die in Wasser gelösten und durch die Vorbehandlung in Lösung gebrachten organischen Stoffe mit Hilfe einer Pumpe 36 in einen Behälter 37 gelangen. Durch die Entwässerung der Suspension werden die bereits gelösten organischen Stoffe abgetrennt und sofort der Biogaserzeugung zugeführt. Es entsteht ein Flüssigkeitsstrom, der eine Konzentration von 20-40 gCSB/l (chemischer Sauerstoffbedarf) und nur noch weniger als 1 % ungelöste Feststoffe aufweist.

Die bei der Fest-Flüssig-Trennung 35 entwässerten Feststoffe, welche nun überwiegend aus unlöslichen Kohlehydraten, Fetten, Proteinen, Cellulose und Lignocellulose bestehen, werden mit Hilfe einer Dickstoffpumpe 38 einem Reaktor für Feststoffhydrolyse 39 zugeführt, die im mesophilen oder im thermophilen Temperaturbereich betrieben werden kann. In der Feststoffhydrolyse werden die Biopolymere durch spezielle hydrolytische Mikroorganismen in lösliche Verbindungen zerlegt. Die Feststoffe werden mit Prozeßwasser vermischt, das mit Hilfe einer Pumpe 40 aus einem Prozeßwasserpuffer 41 entnommen wird, um eine rührfähige Suspension zu erhalten.

Während der Hydrolyse wird die Suspension ständig mit Hilfe einer Pumpe 42 der Fest-Flüssig-Trennung 35 zugeführt, in der die entstandenen Hydrolyseprodukte abgetrennt werden, um sie der Biogas-Produktion, die in einem Methanreaktor 43 stattfindet, zuzuführen.

Die aushydrolysierte Suspension wird über eine Pumpe 44 einer zweiten Fest-Flüssig-Trennung 45 zugeführt und mit dieser entwässert. Die entwässerten Feststoffe 46 können einer aeroben Nachrotte unterworfen und als Bodenverbesserungsmittel eingesetzt werden. Die bei der Fest-Flüssig-Trennung 45 abgetrennte Flüssigkeit wird über eine Pumpe 47 in den Behälter 37 gefördert, in dem sie mit dem Flüssigkeitsstrom aus der Hydrolyse vermischt wird.

Aus dem Behälter 37 werden dem Methanreaktor 43 mittels einer Dosierpumpe 48 kontinuierlich die in Wasser gelösten organischen Stoffe zugeführt, die durch die Methanbakterien in das aus Methan und Kohlendioxid bestehende Biogas 49 umgewandelt werden, das an der Oberseite des Methanreaktors 43 entnommen und einer Verwertung zugeleitet werden kann. Der Ablauf des Methanreaktors 43 wird im Prozeßwasserpuffer 41 gesammelt und zur Auflösung der Abfälle und mittels einer Pumpe 50 zu dem Auflösebehälter 13 zur Auflösung der Abfälle bzw. des Rohmülls gepumpt. Außerdem erfolgt, wie bereits beschrieben durch die Pumpe 40 eine Zuführung vom Prozeßwasser zu der Feststoffhydrolyse 39.

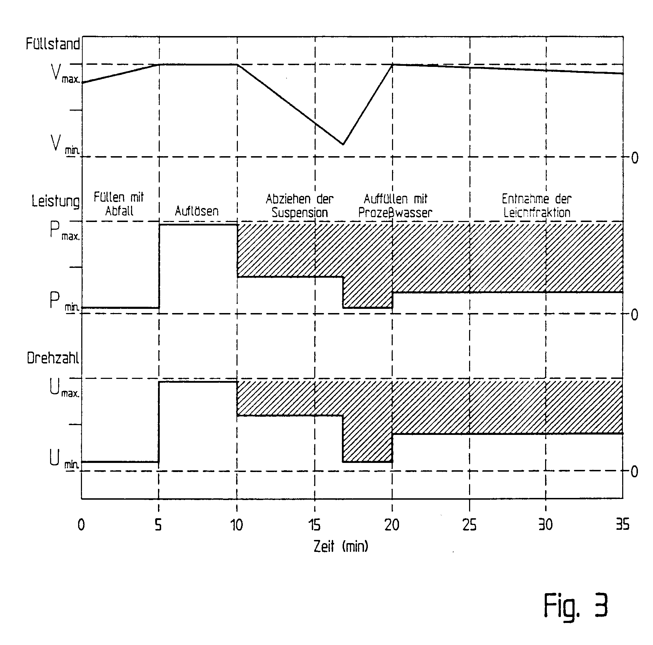

Die bei dem erfindungsgemäßen Verfahren verwendete Vorbehandlungsstufe wird nachfolgend näher erläutert. Ausgegangen wird dabei von einem Auflösebehälter 13, dem eine Charge Rohmüll zugeführt wird. Fig. 3 zeigt eine Sequenz von Betriebsphasen des Laufrades 14, wobei die Drehzahl, die Antriebsleistung und der Füllstand über die Zeit aufgetragen sind. Fig. 3 beschreibt dabei einen auf die Verarbeitung einer Charge bezogenen Arbeitszyklus, wobei sich dieser Zyklus Charge für Charge wiederholt.

Um eine bessere Durchmischung des in dem Auflösebehälter 13 bereits befindlichen Wassers mit den Abfällen zu bewirken, wird der Inhalt des Auflösebehälters 13 durch Drehung des Laufrades 14 bei nur niedriger Drehzahl bewegt. Dabei wird die Größe der Charge so bemessen, daß der Anteil der Feststoffe im Auflösebehälter 13 ungefähr 10 % beträgt. Gemäß dem beispielhaften Zeitablauf von Fig. 3 dauert diese erste Betriebsphase etwa 5 Minuten. Die erforderliche Antriebsleistung ist wegen der geringen Drehzahl des Laufrades während dieser ersten Betriebsphase minimal und liegt an der unteren Betriebsgrenze.

Nach dem vollständigen Einfüllen der Charge in den Auflösebehälter 13 wird die Auflösung und Zerfaserung der organischen Stoffe bei einer hohen Drehzahl des Laufrades 14 durchgeführt. Dabei werden feste organische Stoffe in die Suspension überführt und lösliche Zellinhaltsstoffe, beispielsweise Zucker, in Lösung gebracht. Insbesondere werden die Fettsäuren, die durch Gärungsprozesse während einer mehrtägigen Lagerung von Abfällen durch spontan einsetzende biochemische Prozesse entstehen, in Lösung gebracht. Es entsteht eine Suspension mit einem gelösten CSB von 10-35 g/l. Weitere 80 bis 100 g/l organischer Stoffe liegen ungelöst in Form einer Suspension vor. Aufgrund der durch das Laufrad 14 erzwungenen torroidalen Strömung, die am Boden radial nach außen gerichtet ist und nur eine geringe tangentiale Strömungskomponente aufweist, werden in der Suspension hydraulische Scherkräfte wirksam, die gezielt für das Zerfasern der aufgeweichten biogen-organischen Stoffe eingesetzt werden. Während dieser zweiten Betriebsphase wird aufgrund der hohen Drehzahl des Laufrades 14 eine hohe Antriebsleistung benötigt.

Die Konzentration der löslichen organischen Stoffe in der Suspension erreicht bereits nach wenigen Minuten, beispielsweise gemäß Fig. 3 nach 5 Minuten, einen Sättigungswert. Eine Verlängerung des energieintensiven Auflösens über diesen Zeitpunkt hinaus führt zu keiner weiteren Erhöhung der Konzentration an gelösten Stoffen und birgt zudem die Gefahr einer unerwünschten Zerkleinerung von Fremdstoffen. In der Praxis wurden für den Auflöseprozeß 5 bis 15 Minuten benötigt.

Nach dem Auflösen wird die Suspension durch die Siebplatte 17 mittels der Pumpe 29 in den Rührreaktor 31 gepumpt. Die nicht aufgelösten Bestandteile, vor allem Kunststoffe, Leder und Holz werden von der Siebplatte 17 zurückgehalten. Während des Abpumpens wird die Drehzahl des Laufrades 14 reduziert, um eine unerwünschte Zerkleinerung zu vermeiden und um Energie zu sparen, da, wie auch Fig. 3 zeigt, nur eine verringerte Antriebsleistung erforderlich ist.

Bei niedriger Drehzahl wird anschließend der Auflösebehälter 13 wieder mit Prozeßwasser aufgefüllt, das dem Prozeßwasserpuffer 41 mittels der Pumpe 50 entnommen wird. Als Prozeßwasser wird im Normalbetrieb der Ablauf des Biogasreaktors 43 verwendet, in dem die im Auflösebehälter 13 freigesetzten und in der Feststoffhydrolyse gebildeten gelösten organischen Stoffe zu Methan und Kohlendioxid umgesetzt werden.

Während der Zugabe des Prozeßwassers sammeln sich die Schwerstoffe des Auflösebehälters 13 und werden durch die Bewegung der Flüssigkeit in die geöffnete Schwerstoffschleuse 18 geschwemmt. Eine nicht dargestellte Ablaufsteuerung, die nur in dieser Betriebsphase aktiv ist, sorgt dafür, daß die Schwerstoffschleuse 18 in festgelegten Zeitabständen mit Hilfe des Schiebers 19 geschlossen wird. Der Inhalt der Schwerstoffschleuse 18 wird mit Wasser gespült und durch Öffnen des unteren Schiebers 20 entleert. Nach Schließen des unteren Schiebers 20 und Öffnen des oberen Schiebers 19 ist die Schwerstoffschleuse 18 wieder zur Aufnahme von Schwerstoffen bereit. Die in den Behälter 21 abgegebenen Schwerstoffe werden dort gesammelt und nach Füllen des Containers entsorgt.

In der folgenden Tabelle ist beispielhaft die Zusammensetzung von nach dem beschriebenen Verfahren abgetrennten Schwerstoffen angegeben. Für die Ermittlung der Zusammensetzung wurde der Inhalt der Schwerstoffschleuse nach dem Auflösen einer Charge entnommen, von Hand sortiert und danach getrocknet.

| Fraktion | Trockengewicht kg | Anteil % |
|---|---|---|
| große Steine (>10mm) | 1,24 | 8,3 |
| Steine (<10mm) | 1,72 | 11,5 |
| Gemisch (<10mm) | 7,40 | 49,6 |
| Ton, Ziegel | 1,77 | 11,9 |
| Glas | 1,57 | 10,5 |
| Knochen | 0,43 | 2,9 |
| Batterien | 0,06 | 0,4 |
| Eisenmetalle | 0,59 | 4,0 |
| Buntmetalle | 0,14 | 0,9 |
| Summe | 14,92 | 100,00 |

Die Leichtstoffe werden mit Hilfe der an diesen speziellen Anwendungsfall angepaßten Recheneinrichtung 22 entnommen. Da sich Materialien, aus denen sich die Leichtstoffe zusammensetzen, in ihrer Dichte nur wenig von Wasser unterscheiden, schwimmen sie an die Oberfläche auf oder treiben in der Flüssigkeit. Die Rechenfläche 23 für die Abtrennung der Leichtstoffe kann deshalb mit Hilfe eines elektrischen oder hydraulischen Antriebs soweit in den Auflösebehälter 13 eingefahren werden, daß sie sich vollständig unter der Wasseroberfläche befindet. Die Rechenfläche 23 füllt den zwischen Laufrad 14 und Wand des Auflösebehälters 13 verbleibenden freien Querschnitt des Auflösebehälters 13 möglichst vollständig aus und besteht aus einem Rahmen mit Querstreben, an denen im Abstand von 5 bis 7 cm die Zinken 23' mit einer Länge von 10 bis 15 cm senkrecht angebracht sind.

Die Bewegungsgeometrie der Recheneinrichtung 22 ist so gestaltet, daß der Rechen aus dem Auflösebehälter 13 ausgefahren werden kann, ohne daß die anhängenden Leichtstoffe durch das Bewegen abfallen. Das Abwerfen der Leichtstoffe wird erst außerhalb des Auflösebehälters 13 durch Schwenken der Rechenfläche 23 in eine waagrechte Position gezielt eingeleitet. Die Bewegung der Recheneinrichtung 22 wird von einer nicht dargestellten vollautomatischen Ablaufsteuerung kontrolliert. Sie wird erst abgeschaltet, wenn sich nur noch unbedeutende Mengen der Leichtstoffe in den Zinken 23' verfangen.

In der folgenden Tabelle ist die typische Zusammensetzung der mit dem beschreibenen Verfahren abgetrennten Leichtstoffe angegeben:

| Sortierfraktion | Massenanteil feucht | | Massenanteil trocken | | Wassergehalt (%) | Glühverlust (%) |
|---|---|---|---|---|---|---|
| | (kg) | (%) | (kg) | (%) | | |
| Kunststoffe | 70,98 | 26,1 | 36,85 | 31,5 | 48,4 | 88,2 |
| Textilien | 43,50 | 16,0 | 12,67 | 10,8 | 70,8 | 87,0 |
| nativ Organisches | 13,05 | 4,8 | 2,76 | 2,4 | 79,8 | 89,4 |
| Holz, Knochen Inertes | 27,02 | 9,9 | 14,05 | 12,0 | 47,9 | |
| Glas | 0,46 | 0,2 | 0,46 | 0,4 | 10,7 | |
| Hartplastik | 7,56 | 2,7 | 7,14 | 6,1 | 5,2 | |
| Metalle | 4,12 | 1,5 | 3,45 | 2,9 | 16,6 | |
| Feinanteil | 50,37 | 18,5 | 21,42 | 18,3 | 57,9 | 58,5 |
| nichtsortierbarer Rest | 54,95 | 20,2 | 18,19 | 15,6 | 66,9 | 80,0 |

Nach der Entnahme der Leichtstoffe steht der Auflösebehälter 13 wieder bereit für die Aufnahme einer neuen Charge des Abfalls. Die Leichtstoffe werden direkt oder nach Aufbereitung in dem Shredder 25 und auf dem Vibrationssortierer 26 in den Container 28 abgeworfen.

Die Drehzahl des Laufrades 14 wird, wie Fig. 3 zu entnehmen ist, nach dem Abziehen der Suspension wieder deutlich nahe der minimalen Drehzahl für die erste Phase des Auffüllens mit Prozeßwasser auf eine erste Zwischendrehzahl verringert, wodurch auch die erforderliche Antriebsleistung deutlich sinkt. Während des zweiten Abschnitts des Auffüllens mit Prozeßwasser, der wiederum etwa 5 Minuten dauert, und während der sich daran anschließenden Entnahme der Leichtfraktion mittels der Recheneinrichtung 22 wird die Drehzahl des Laufrades 14 auf einer zweiten Zwischendrehzahl gehalten, die zwischen der ersten Zwischendrehzahl während des Abziehens der Suspension und der Anfangsdrehzahl beim Füllen mit Abfall liegt. Die Energieeinsparung im Vergleich zum Betrieb ohne eine entsprechende Steuerung der Drehzahl läßt sich anhand der schraffierten Fläche in Fig. 3 abschätzen. Sie hängt im wesentlichen vom Anteil der Leichtfraktion ab und wird umso größer, je größer die Zeit ist, die für die vollständige Entnahme der Leichtfraktion benötigt wird. Beispielsweise beträgt die Leistungsaufnahme des Antriebs des Laufrades 14 in einem Auflösebehälter 13 mit einem Fassungsvolumen von 4 $m^3$ etwa 10 bis 15 $kW/m^3$ für die Betriebsphase des Auflösens, während für das Durchmischen bei der Entnahme der Leichtstoffe nur 2,5 bis 3 $kW/m^3$ benötigt werden.

Die folgende Tabelle zeigt die Leistungsfähigkeit des beschriebenen Verfahrens. Es wurden die Mengen der abgeschiedenen Fremdstoffe (Schwer- und Leichtstoffe) verglichen mit den Mengen, die bei der Handsortierung desselben Abfalls erhalten wurden. Die Ausbringungsgrade liegen für alle Fraktionen nahe bei 1. Das bedeutet, daß die Fremdstoffe mit hohem Wirkungsgrad durch das beschriebene Verfahren abgetrennt werden können.

| Fraktion | Glas (kg) | Metalle (kg) | Mineral-stoffe (kg) | Kunst-stoffe (kg) | Textilien (kg) | Holz (kg) |
|---|---|---|---|---|---|---|
| Anteil in: | | | | | | |
| Vorsortierung | 3,4 | 3,3 | 0,8 | 0,7 | | |
| Schwerstoffe | 0,8 | 0,4 | 2,6 | | | |
| Rechengut | 0,1 | 0,8 | | 10,8 | 3,1 | 3,4 |
| Summe | 4,3 | 4,5 | 3,4 | 11,5 | 3,1 | 3,4 |
| Anteil in der Handsortierung | 4,7 | 4,5 | 3,9 | 9,2 | 2,6 | 3,8 |
| Ausbringungsgrad | 0,91 | 1,0 | 0,87 | 1,25 | 1,2 | 0,89 |

Unter Ausbringungsgrad versteht man das Verhältnis zwischen dem Gewicht der im Verfahren mechanisch abgetrennten Fraktionen zum Gewicht derselben, aber durch Handsortierung gewonnenen Fraktionen. Die beiden Chargen für den Vergleich wurden durch repräsentative Probenahme aus derselben Lieferung von Abfallstoffen entnommen. Die Abweichungen vom Wert 1, die innerhalb eines zulässigen Toleranzbereichs von 20% liegen, sind durch die unvermeidliche Inhomogenität der Abfälle bedingt.

In der folgenden Tabelle sind für die thermisch-chemische Behandlung der Abfallarten "Naßmüll" und "Biomüll" in den Rührreaktor 31 typische Ergebnisse dargestellt. Die Wirkung der thermisch-chemischen Behandlung führt zu einer weiteren Auflösung der organischen Trockensubstanz (oTS) und der Anteil an "Neutral Detergent Fiber", der ein Maß für die biochemisch leicht umsetzbaren Stoffe darstellt, erhöht sich im Vergleich zum ursprünglichen Abfall.

| Substrat | NaOH-Dosierung gNaOH/kgTR | Auflösung der oTS | NDF-Gehalt | pH-Wert |
|---|---|---|---|---|
| Naßmüll | 15-50 | 17 % | 55 % | 7-9 |
| Biomüll | 14-45 | 14 % | 45 % | 6,3 |

Der Umfang der durch die thermisch-chemische Behandlung bewirkten Auflösung kann durch Wahl der Behandlungstemperatur spezifiziert werden. In der folgenden Tabelle sind typischen Werte für die Erhöhung der gelösten organischen Stoffe, gemessen als CSB und Trockensubstanz, für verschiedene Betriebstemperaturen aufgeführt.

| | Temperatur °C | Erhöhung des gelösten CSB % | Erhöhung der gelösten oranischen Trockensubstanz % |
|---|---|---|---|
| Naßmüll | 65 | 18 | 26 |
| | 70 | 50 | 71 |
| Biomüll | 65 | 6 | 1 |
| | 70 | 23 | 54 |

Durch die Entwässerung der Suspension in der Fest-Flüssig-Trennung 35 werden die bereits gelösten organischen Stoffe abgetrennt und sofort der Biogas-Erzeugung zugeführt. Es entsteht ein Flüssigkeitsstrom, der eine Konzentration von 10 bis 40 gCSB/l und nur noch weniger als 1% ungelöste Feststoffe aufweist.

Typische Werte für die durch die Hydrolyse in dem Hydrolysereaktor 39 erreichte spezifische Produktion des gelösten organischen Trockenrückstands (oTR) und die spezifische Abnahme der organischen Trockensubstanz (oTS) sind in der folgenden Tabelle zusammengestellt.

| Verdünnungsrate (1/d) | Hydrolysezeit (h) | spezifische Produktion gelöste oTR (%) | spezifische Abnahme oTS (%) |
|---|---|---|---|
| 1,5 | 49,5 | 19,34 | 64,25 |
| 1,5 | 70,5 | 45,33 | 85,89 |
| 1,5 | 49,3 | 66,78 | 93,91 |
| 2,0 | 23,2 | 16,50 | 18,04 |
| 2,0 | 44,5 | 17,23 | 43,47 |
| 2,0 | 47,0 | 16,42 | 74,84 |
| 1,0 | 42,5 | 58,50 | 76,89 |
| 1,0 | 20,0 | 55,14 | 66,62 |

Zur Abtrennung der Leichtstoffe kann alternativ zu der beschriebenen Recheneinrichtung 22 eine Reinigungskammer 51 vorgesehen sein, die sich entweder unmittelbar an den Auflösebehälter 13 anschließt oder über eine Leitung mit diesem verbunden ist. Die Reinigungskammer 51 besteht aus einem geschlossenen Behälter, der mit Hilfe eines Schiebers 52 bei Bedarf zeitweise mit dem Auflösebehälter 13 verbunden werden kann.

Die Leichtstoffe werden aus dem Auflösebehälter 13 nach dem Abziehen der Rohsuspension mit Hilfe von Prozeßwasser in die Reinigungskammer 51 nach Öffnen des Schiebers 52 geschwemmt. Die Reinigungskammer 51 ist mit einem auf einer horizontalen Achse angebrachten annähernd vertikal orientierten Laufrad 53 versehen. Durch eine hinter dem Laufrad 53 angebrachte Sieblochung 54, die in Form von Siebschlitzen ausgebildet sein kann, wird mit Hilfe einer Pumpe 55 eine dünne Fasersuspension abgepumpt. Bei Bedarf kann mit der Pumpe 56 anschließend erneut Prozeßwasser als Spülwasser zugeführt und dann eine weitere Fasersuspension abgepumpt werden. Die aus der Reinigungskammer 51 abgepumpte verdünnte Suspension bildet einen Teil der Wasservorlage für das Auflösen der nächsten Charge in dem Auflösebehälter 13. Die Leichtstoffe können durch einen Schieber 57 aus der Reinigungskammer 51 entfernt und einem Behälter 58 zum Sammeln und zum Abtransport der Leichtstoffe zugeführt werden.

Bei Verwendung der Reinigungskammer 51 ergibt sich ebenso wie bei der Verwendung der Recheneinrichtung gemäß Figur 4 ein niedriger Energieverbrauch. Der Antrieb für das Laufrad 53 benötigt dieselbe Energie wie der des Laufrades 14 des Auflösebehälters 13, der bei der ersten alternativen Ausführungsform während des Betriebs der Recheneinrichtung weiterläuft, während bei Verwendung der Reingungskammer 51 gemäß der alternativen Ausgestaltung das Laufrad 14 des Auflösebehälters 13 nicht zum Austrag von Leichtstoff betrieben werden muß.

Der Einsatz der Reinigungskammer 51 ist vor allem für solche Abfallarten günstig, bei denen die organischen Faserstoffe innig mit den Leichtstoffen derart verbunden sind, daß durch der Entnahme mit der Recheneinrichtung 22 die angestrebte Trennwirkung nicht erreicht werden kann.

**Patentansprüche**

1. Verfahren zur Aufbereitung von Stoffgemischen für die anaerobe Vergärung der darin enthaltenen biogen-organischen Stoffe, die für eine anaerobe Vergärung geeignet sind, insbesondere von Biomüll, Naßmüll, Restmüll und Gewerbeabfällen, bestehend aus den folgenden Verfahrensschritten:
   (a) in einer Vorbehandlungsstufe wird eine leicht entwässerbare Suspension der biogen-organischen Stoffe, die für eine anaerobe Vergärung geeignet sind, durch eine mechanische Aufbereitung mit selektiver Auflösung und Zerfaserung der biogenorganischen Stoffe, die für eine anaerobe Vergärung geeignet sind, hergestellt, wobei die Einwirkung strömungs-mechanischer Kräfte derart gesteuert wird, daß die Fremdstoffe, die für eine anaerobe Vergärung ungeeignet sind, vor ihrer Abtrennung keiner nennenswerten Zerkleinerungsbeanspruchung unterliegen, wobei die Fremdstoffe, die für eine anaerobe Vergärung ungeeignet sind, abgetrennt werden; und

(b) die biogen-organischen Stoffe, die für eine anaerobe Vergärung geeignet sind, werden zu Biogas mittels eines anaeroben Vergärungsprozesses sowie zu kompostähnlichen Reststoffen umgewandelt.

2. Verfahren nach Anspruch 1,
dadurch gekennzeichnet,
daß in der Vorbehandlungsstufe die Suspension aus gelösten und zerfaserten biogen-organischen Stoffen, die für eine anaerobe Vergärung geeignet sind, diskontinuierlich abgezogen wird und die verbleibenden Fremdstoffe, die für eine anaerobe Vergärung ungeeignet sind, mit Wasser, vorzugsweise Prozeßwasser, derart aufgeschlämmt werden, daß anschließend die Schwerstoffe abtrennbar und auf- bzw. in der Fremdstoffsuspension schwimmende Leichtstoffe entnehmbar sind.

3. Verfahren nach Anspruch 1 oder 2,
dadurch gekennzeichnet,
daß die mechanische Aufbereitung in einem Auflösebehälter mit einem drehbaren Laufrad durchgeführt wird, wobei die Drehzahl des drehbaren Laufrades so gewählt wird, daß eine schonende Behandlung der Fremdstoffe, die für eine anaerobe Vergärung ungeeignet sind, bewirkt wird.

4. Verfahren nach Anspruch 3,
dadurch gekennzeichnet,
daß das Laufrad während der Auflösung und Zerfaserung der biogen-organischen Stoffe, die für eine anaerobe Vergärung geeignet sind, mit hoher Geschwindigkeit und während der Abtrennung der Fremdstoffe, die für eine anaerobe Vergärung ungeeignet sind, mit verringerter Geschwindigkeit betrieben wird.

5. Verfahren nach einem der vorangehenden Ansprüche,
dadurch gekennzeichnet,
daß bei der Aufbereitung wenigstens der Beginn des Auflösevorgangs im Auflösebehälter in Zusammenhang mit einer torroidalen bzw. schleifenförmigen Strömung des Inhalts des Auflösebehälters durchgeführt wird.

6. Verfahren nach einem der Ansprüche 2 bis 5,
dadurch gekennzeichnet,
daß die entnommenen Leichtstoffe einer zusätzlichen Reinigungsbehandlung unterworfen werden, wobei das dabei entfernte Material in den Auflösebehälter zugeführt wird.

7. Verfahren nach einem der vorangehenden Ansprüche,
dadurch gekennzeichnet,
daß in der Vorbehandlungsstufe eine Suspension hergestellt wird, deren Anteil an biogen-organischen Stoffen, die für eine anaerobe Vergärung geeignet sind, bezogen auf die Trockenmasse größer ist als in den Stoffgemischen, die für die Aufbereitung vorgesehen sind.

8. Verfahren nach einem der vorangehenden Ansprüche,
dadurch gekennzeichnet,
daß die Suspension nach der Vorbehandlungsstufe durch Zugabe von Chemikalien auf einen pH-Wert im alkalischen Bereich eingestellt, auf 40 - 90°C, vorzugsweise auf 60 - 70°C, erwärmt und bis zu 12 Stunden auf dieser Temperatur gehalten wird.

9. Verfahren nach einem der vorangehenden Ansprüche,
dadurch gekennzeichnet,
daß die Suspension beim Verfahrensschritt (b) durch eine Fest-Flüssig-Trennstufe in einen die wasserlöslichen, biologischen Stoffe enthaltenen Flüssigkeitsstrom und in einen Feststoffstrom mit weniger als 50 Gew.-%, vorzugsweise 20 - 50 Gew.-%, Trockenmasse aufgeteilt wird, wobei der Flüssigkeitsstrom anschließend einer Methangärung zugeführt wird, und wobei der Feststoffstrom einer anaeroben Feststoff-Hydrolyse im meso- oder thermophilen Temperaturbereich ausgesetzt und das dabei gewonnene Hydrolysat der Methangärung zugeführt wird.

10. Verfahren nach einem oder mehreren der vorhergehenden Ansprüche,
dadurch gekennzeichnet,

daß die Suspension beim Verfahrensschritt (b) durch eine Fest-Flüssig-Trennstufe in einen die wasserlöslichen, biologischen Stoffe enthaltenden Flüssigkeitsstrom und in einen Feststoffstrom aufgeteilt wird, wobei die Feststoffe einer aeroben Behandlung zugeführt werden.

11. Verfahren nach einem der Ansprüche 1 - 9,
dadurch gekennzeichnet,
daß die faserhaltigen Feststoffe in einer mindestens einstufigen Feststoffhydrolyse in lösliche organische Stoffe umgewandelt werden, wobei die Hydrolyse durch von außen zugegebene Enzyme erfolgt, die entweder in einem parallel geschalteten Reaktor produziert werden oder durch Beimpfung der Feststoffe mit speziellen Mikroorganismen entstehen, und wobei die gelösten Stoffe kontinuierlich oder diskontinuierlich abgetrennt und der Methangärung zugeführt werden.

12. Verfahren nach Anspruch 11,
dadurch gekennzeichnet,
daß durch eine zusätzliche mechanische, chemische oder thermische Behandlung oder deren Kombination die Hydrolysierbarkeit der organischen Faserstoffe erhöht und die Abtrennung der nicht hydrolysierbaren Feststoffe von den gelösten organischen Stoffen erleichtert wird.

13. Verfahren nach einem der Ansprüche 2 bis 12,
dadurch gekennzeichnet,
daß die entnommenen Leichtstoffe zerkleinert und von noch anhaftenden Faserresten gereinigt werden.

14. Verfahren nach Anspruch 13,
dadurch gekennzeichnet,
daß die Leichtstoffe mit Hilfe einer Preßvorrichtung im Volumen reduziert und entwässert werden, wobei das Preßwasser wieder in den Auflösebehälter zurückgeführt wird.

15. Verfahren nach einem der vorhergehenden Ansprüche, dadurch gekennzeichnet, daß die Einwirkung strömungsmechanischer Kräfte in Abhängigkeit von der Feststoffkonzentration gesteuert wird.

16. Vorrichtung zur Durchführung des Verfahrens nach Anspruch 1, mit einem ein drehbares Laufrad (14) aufweisenden Auflösebehälter (13), dadurch gekennzeichnet, daß für das Laufrad (14) eine Drehzahlsteuerung vorgesehen ist, mit deren Hilfe die Einwirkung strömungsmechanischer Kräfte derart gesteuert wird, daß die Fremdstoffe, die für eine Vergärung ungeeignet sind, vor ihrer Abtrennung keiner nennenswerten Zerkleinerungsbeanspruchung unterliegen, und daß das Laufrad (14) zur Schaffung einer torroidalen bzw. schleifenförmigen Strömung der Suspension in dem Auflösebehälter (13) ausgebildet ist.

17. Vorrichtung nach Anspruch 16, dadurch gekennzeichnet, daß unterhalb des vertikal angeordneten Laufrades (14) eine Siebplatte (17) für das Abziehen der Suspension aus gelösten und zerfaserten biogen-organischen Stoffen, die für eine anaerobe Vergärung geeignet sind, vorgesehen ist.

18. Vorrichtung nach Anspruch 16 oder 17,
dadurch gekennzeichnet,
daß dem Auflösebehälter (13) zum Abschöpfen bzw. Trennen der Leichtstoffe eine Recheneinrichtung (22) zugeordnet ist, die eine Rechenfläche (23) mit Rechenzinken (23') aufweist, welche zum Festhalten der Leichtstoffe vertikal stellbar und unter den Flüssigkeitsspiegel (24) im Auflösebehälter (13) eintauchbar ist, wobei die Leichtstoffe durch Ausschwenken der Rechenvorrichtung (22) aus dem Auflösebehälter (13) entfernbar und durch Kippen der Rechenfläche (23) abwerfbar sind.

19. Vorrichtung nach Anspruch 18,
dadurch gekennzeichnet,
daß für die mit der Recheneinrichtung (22) entfernten Leichtstoffe eine Zerkleinerung in einem Shredder (25) und ein anschließendes Abwaschen noch anhaftender Faserreste auf einem Vibrationssortierer (26) vorgesehen ist.

20. Vorrichtung nach einem oder mehreren der vorhergehenden Ansprüche,
dadurch gekennzeichnet,

daß die Leichtstoffe zusammen mit Wasser in eine Reinigungskammer mit einem vertikalen Laufrad überführbar sind, aus der das Wasser und noch an den Leichtstoffen anhaftendes Fasermaterial durch ein Sieb abziehbar und in den Auflösebehälter (13) zurückpumpbar ist.

21. Vorrichtung nach einem oder mehreren der vorhergehenden Ansprüche,
dadurch gekennzeichnet,
daß der Auflösevorgang im Auflösebehälter (13) bei einer Temperatur zwischen 40 - 90 °C, vorzugsweise bei 60 - 70 °C, durchführbar ist, wobei für die Erwärmung der Suspension vorgewärmtes Prozeßwasser vorgesehen ist.

**Claims**

1. A process of treating mixtures of substances for anaerobic digestion of biogenic-organic substances contained therein which are suitable for anaerobic digestion, in particular of organic waste, wet waste, residual waste and commercial waste, consisting of the following process steps:

   (a) preparing an easily dewaterable suspension of biogenic-organic substances which are suitable for anaerobic digestion by mechanical treatment comprising selectively dissolving and defibrating said biogenic-organic substances which are suitable for anaerobic digestion in a pretreatment step wherein in the action of flow-dynamical forces is controlled so that the foreign substances which are not suitable for an anaerobic digestion are not subjected to an appreciable disintegration before they are removed, wherein the foreign substances which are not suitable for an anaerobic digestion are separated; and

   (b) converting said biogenic-organic substances which are suitable for an anaerobic digestion to biogas by an anaerobic digestion process and to compost-like residual substances.

2. The process according to claim 1,
   **characterized in**
   that in the pretreatment step the suspension containing dissolved and defibrated biogenic-organic substances which are suitable for an anaerobic digestion is intermittently withdrawn, and the remaining foreign substances which are not-suitable for an anaerobic digestion are slurred with water, preferably with process water, so that the heavy solids can subsequently be separated and lightweight substances which have risen to the surface of the suspension containing foreign substances or which floated in that suspension can be withdrawn.

3. The process according to claim 1 or 2,
   **characterized in**
   that the mechanical treatment is performed in a pulping vessel which contains a rotating impeller, wherein the speed of the impeller is selected so that a gentle treatment of the foreign substances which are not suitable for an anaerobic digestion is effected.

4. The process according to claim 3,
   **characterized in**
   that the impeller is operated during dissolving and defibrating of the biogenic-organic substances which are suitable for an anaerobic digestion with a high speed and during separating of the foreign substances which are not suitable for an anaerobic digestion with a reduced speed.

5. The process according to any of the preceding claims,
   **characterized in**
   that during treatment at least the beginning of the dissolving operation is performed in the pulping vessel in cooperation with a torroidal or loop-shaped flow of the content of the pulping vessel.

6. The process according any of claims 2 to 5,
   **characterized in**
   that the withdrawn lightweight substances are subjected to an additional cleaning treatment wherein the material withdrawn hereby is turned to the pulping vessel.

7. The process according to any of the preceding claims,
   **characterized in**

that a suspension is formed in the pretreating step, the ratio of biogenic-organic substances in that suspension which are suitable for an anaerobic digestion relativ to its dry solid content exceeds said ratio in said mixture of substances to be treated.

8. The process according to any of the preceding claims,
   **characterized in**
   that the suspension is adjusted by an addition of chemicals to a pH-value in the alkaline range and is heated to a temperature of 40 to 90°C, preferably to a temperature of 60 to 70°C, and is held at that temperature for up to twelve hours.

9. The process according to any of the preceding claims,
   **characterized in**
   that in the process step (b) the suspension is divided in a solid-liquid separating step into a liquid stream, which contains the water-soluble organic substances, and a solids-loaden stream with a dry solids content of less than 50 % per weight, preferably 20 to 50 % per weight, wherein the liquid stream is subsequently subjected to a methan formation, and wherein the solids-loaden stream is subjected to an anaerobic hydrolysis of solids in the mesophilic of thermophilic temperature range, and the resulting hydrolysate is subjected to a methan formation.

10. The process according to any of the preceding claims,
    **characterized in**
    that in the process step (b) the suspension is divided in a solid-liquid separating stage into a liquid stream which contains the water-soluble organic substances and a solids-loaden stream, wherein the solids are conveyed to an aerobic treatment.

11. The process according to any of claims 1 to 9,
    **characterized in**
    that the fibre-containing solids are converted to soluble organic substances in at least a one stage solids hydrolysis, wherein said hydrolysis is effected by added extraneous enzymes which are produced either in the reactor which is connected in parallel or as a result of an inoculation of the solids with special microorganisms, and the dissolved substances are continously or intermittently removed and supplied to the methan formation.

12. The process according to claim 11,
    **characterized in**
    that an additional mechanical, chemical or thermal treatment or a combination thereof is performed to increase the hydrozability of the organic fibre substances and to facilitate the separation of the non-hydrolyzable solids from the dissolved organic substances.

13. The process according to any claims 2 to 12,
    **characterized in**
    that the lightweight substances withdrawn are reduced in size and cleaned to remove any still adhering residual fibre substances.

14. The process according to claim 13,
    **characterized in**
    that the lightweight substances are compacted and dewatered by compacting means, and the water which has been squeezed out is returned to the pulping vessel.

15. The process according to any of the preceding claims,
    **characterized in**
    that the action of flow-dynamical forces is controlled in dependence on the solids concentration.

16. Apparatus for performing the process according to claim 1, comprising a pulping vessel (13) which contains a rotating impeller (14)
    **characterized in**
    that the impeller (14) comprises an element to control the speed thereof to control the action of flow-dynamical forces so that the foreign substances which are not suitable for digestion are not subjected to an appreciable desintegration before separating the foreign substances, and that the impeller (14) is designed to generate a torroidal and/or loop-shaped flow in the suspension in the pulping vessel (13).

**17.** The apparatus according to claim 16,
**characterized in**
that a sieve plate (17) for the removal of the suspension of dissolved and defibrated biogenic organic substances which are suitable for an anaerobic digestion is provided below the vertically oriented impeller (14).

**18.** The apparatus according to claim 16 or 17,
**characterized in**
that a raking device (22) for skimming off or separating lightweight substances is associated with the pulping vessel (13) and comprises a tine carrier (23) which is provided with tines (23') and is vertically adjustable so that the tines can catch the lightweight substances and is adapted to be immersed under the liquid level (24) in the pulping vessel (13), and the raking device (22) is removable out of the pulping vessel (13) so that the lightweight substances can be removed, and the tine carrier (23) adapted to be tilted to throw off the lightweight substances.

**19.** The apparatus according to claim 18,
**characterized in**
that the shredder (25) is provided for the size reduction of the lightweight substances which have been removed by the raking device (22) and is succeeded by a vibratory sorting (26) for rinsing off any residual fibre material which is still adhering.

**20.** The apparatus according to any of the preceding claims,
**characterized in**
that the lightweight substances are adapted to be transferred together with the water into a cleaning compartment which contains a vertically oriented impeller, and the water and any fibre material still adhering to the lightweight substances are adapted to be withdrawn from said cleaning compartment through a sieve and to be pumped back to the pulping vessel.

**21.** The apparatus according to any of the preceding claims,
**characterized in**
that the dissolving operation in the pulping vessel (13) is adapted to be performed at a termperature between 40 and 90°C, preferably at 60 to 70°C, and preheated process water is used to heat the suspension.

**Revendications**

**1.** Procédé pour préparer des mélanges de substances pour la fermentation anaérobie des substances biogènes-organiques contenues dans ces mélanges, et qui conviennent pour une fermentation anaérobie, notamment de déchets organiques, de déchets humides et de résidus industriels, comprenant les étapes opératoires suivantes :
a) dans une étape de prétraitement, on fabrique une suspension légèrement déshydratée des substances biogènes-organiques, qui conviennent pour une fermentation anaérobie, au moyen d'un traitement mécanique avec une dissolution sélective et une trituration des substances biogènes-organiques, qui conviennent pour une fermentation anaérobie, l'action de forces exercées par les fluides étant commandée de telle sorte que les substances étrangères, qui ne conviennent pas pour une fermentation anaérobie ne sont soumises, avant leur séparation, à aucune contrainte notable de fragmentation, grâce à quoi les substances étrangères, qui ne conviennent pas pour une fermentation anaérobie, sont séparées; et
b) les substances biogènes-organiques, qui conviennent, pour une fermentation au aérobie, sont converties en un biogaz au moyen d'un processus de fermentation anaérobie, ainsi qu'en des matières résiduelles semblables à du compost.

**2.** Procédé selon la revendication 1, caractérisé en ce que lors de l'étape de traitement préalable, on prélève de façon discontinue la suspension formée de substances biogènes-organiques dissoutes et triturées, qui sont appropriées pour une fermentation anaérobie, et on met en suspension les substances étrangères restantes, qui ne sont pas appropriées pour une fermentation anaérobie, avec de l'eau, de préférence de l'eau de traitement, de telle sorte qu'on peut ensuite séparer les substances lourdes et retirer les substances légères qui flottent sur ou dans la suspension de substances étrangères.

3. Procédé selon la revendication 1 ou 2, caractérisé en ce que le traitement mécanique est exécuté dans un récipient de dissolution comportant une roue rotative, la vitesse de rotation de la roue rotative étant choisie de telle sorte qu'on obtient un traitement avec ménagement de substances étrangères, qui ne conviennent pas pour une fermentation anaérobie.

4. Procédé selon la revendication 3, caractérisé en ce que la roue est entraînée avec une vitesse élevée pendant la dissolution et la trituration des substances biogènes-organiques, qui sont appropriées pour une fermentation anaérobie, et avec une vitesse réduite pendant la séparation des substances étrangères, qui ne sont pas appropriées pour une fermentation anaérobie.

5. Procédé selon l'une des revendications précédentes, caractérisé en ce que, lors de la préparation, au moins le début du processus de dissolution dans le récipient de dissolution est exécuté en liaison avec un écoulement toroïdal ou en boucle du contenu du récipient de dissolution.

6. Procédé selon l'une des revendications 1 à 5, caractérisé en ce que les substances légères prélevées sont soumises à un traitement supplémentaire d'épuration, le matériau retiré étant envoyé dans le récipient de dissolution.

7. Procédé selon l'une des revendications précédentes, caractérisé en ce que lors de l'étape de traitement préalable, on fabrique une suspension, dont le pourcentage en substances biogènes-organiques, qui sont appropriées pour une fermentation anaérobie, par rapport à la masse sèche est supérieur au pourcentage de ces substances dans les mélanges de substances qui sont prévus pour la préparation.

8. Procédé selon l'une des revendications précédentes, caractérisé en ce qu'avant l'étape de prétraitement, la suspension est réglée, par adjonction de produits chimiques, sur une valeur du pH dans le domaine alcalin, est chauffée à 40-90°C et de préférence à 60-70°C et est conservée à cette température pendant une durée atteignant jusqu'à 12 heures.

9. Procédé selon l'une des revendications précédentes, caractérisé en ce que, lors de l'étape opératoire (b), la suspension est répartie, sous l'effet d'une étape de séparation substances solides - substances liquides, en un écoulement liquide, qui contient des substances biologiques solubles à l'eau, et en un courant de substances solides possédant une masse sèche correspondant à moins de 50 % en poids et de préférence comprise entre 20 et 50 % en poids, l'écoulement liquide étant ensuite soumis à une fermentation méthanique, tandis que le courant de substances solides est soumis à une hydrolyse anaérobie des substances solides dans la gamme mésophile ou thermophile de températures et l'hydrolysat obtenu est soumis à la fermentation méthanique.

10. Procédé selon une ou plusieurs des revendications précédentes, caractérisé en ce que lors de l'étape opératoire (b), la suspension est répartie, sous l'effet d'une étape de séparation substances solides - substances liquides, en un écoulement liquide, contenant les substances biologiques solubles à l'eau, et en un courant de substances solides, les substances solides étant soumises à un traitement aérobie.

11. Procédé selon l'une des revendications 1-9, caractérisé en ce que les substances solides contenant des fibres sont converties, lors d'au moins une hydrolyse des substances solides exécutées en une seule étape, en des substances organiques solubles, l'hydrolyse étant exécutée par des enzymes ajoutées de l'extérieur et qui sont produites soit dans un réacteur branché en parallèle, soit par inoculation de micro-organismes spéciaux dans les susbstances solides, et les substances dissoutes étant séparées de façon continue ou discontinue et étant soumises à une fermentation méthanique.

12. Procédé selon la revendication 11, caractérisé en ce qu'à l'aide d'un traitement mécanique, chimique ou thermique supplémentaire ou au moyen de la combinaison de tels traitements, on augmente la capacité d'hydrolyse des substances fibreuses organiques et on facilite la séparation des substances solides non hydrolysables, par rapport aux substances organiques dissoutes.

13. Procédé selon l'une des revendications 2 à 12, caractérisé en ce que les substances légères prélevées sont fragmentées et sont séparées des restes de fibres, qui adhèrent encore à ces substances.

14. Procédé selon la revendication 13, caractérisé en ce que le volume des substances légères est réduit et ces substances légères sont déshydratées à l'aide d'un dispositif de pressage, l'eau de pressage étant à nouveau renvoyée au récipient de dissolution.

15. Procédé selon l'une des revendications précédentes, caractérisé en ce que l'action de forces exercées par les fluides est commandée en fonction de la concentration de substances solides.

16. Procédé pour la mise en oeuvre du procédé selon la revendication 1, comportant un récipient de dissolution (13), qui possède une roue rotative (14), caractérisé en ce que pour la roue (14) il est prévu une unité de commande de la vitesse de rotation, à l'aide de laquelle l'action de forces fluidiques est commandée de telle, sorte que les substances étrangères, qui ne conviennent pas pour une fermentation, ne sont soumises, avant leur séparation, à aucune contrainte notable de fragmentation, et que la roue (14) est agencée de manière à créer un écoulement toroïdal ou en boucle de la suspension dans le récipient de dissolution (13).

17. Dispositif selon la revendication 16, caractérisé en ce qu'une plaque formant tamis (17) est prévue au-dessous de la roue (14) disposée verticalement, pour retirer la suspension à partir des substances biogènes-organiques dissoutes et triturées, qui conviennent pour une fermentation anaérobie.

18. Dispositif selon la revendication 16 ou 17, caractérisé en ce qu'au récipient de dissolution (13) est associé, pour l'épuisement ou la séparation des substances légères, un dispositif à râteau (22), qui comporte une surface de râteau (23) comportant des dents (23'), qui est réglable verticalement pour retenir les substances légères et peut être immergé dans le récipient de dissolution (13) au-dessous du niveau du liquide (24), les substances légères pouvant être retirées par basculement du dispositif à râteau (22) hors du récipient de dissolution (13) et être éjectées sous l'effet d'un basculement de la surface du râteau (23).

19. Dispositif selon la revendication 18, caractérisé en ce que pour les substances légères retirées à l'aide du dispositif à râteau (22), il est prévu une fragmentation dans un déchiqueteur (25), et une élimination ultérieure par lavage de résidus de fibres, qui adhèrent encore, sur un dispositif de tri vibrant (26).

20. Dispositif selon une ou plusieurs des revendications précédentes, caractérisé en ce que les substances légères peuvent être transférées, conjointement avec de l'eau, dans une chambre de nettoyage comportant un rotor vertical et d'où le matériau fibreux, qui adhère encore aux substances légères, peuvent être retirées au moyen d'un tamis et être renvoyées par pompage dans le récipient de dissolution (13).

21. Dispositif selon une ou plusieurs des revendications précédentes, caractérisé en ce que le processus de dissolution dans le récipient de dissolution (13) peut être exécuté à une température comprise entre 40 et 90°C et de préférence entre 60 et 70°C, de l'eau de traitement préchauffée étant prévue pour le chauffage de la suspension.

Rohmüll → **SackaufreiBer / Magnetabscheider** → Eisenmetalle

**SackaufreiBer**

**Magnetabscheider**

ProzeBwasser → **Auflösebehälter** → Leichtstoffe

→ Schwerstoffe

Suspension | Suspension

**Verwertung des Biogases**

**physik.-chemische Behandlung der Suspension**

Suspension

Flüssigstrom → **Fest-Flüssig-Trennung 1**

**Methanreaktor**

Feststoff | Feststoff

**physik.-chemische Behandlung der Feststoffe**

Feststoff

Verdünnungs-wasser → **Hydrolyse-reaktor**

Suspension

ÜberschuBwasser

**Fest-Flüssig-Trennung 2**

Reststoff

**Behandlung des ÜberschuBwassers**

**Nachbehandlung der organischen Reststoffe**

Fig. 1

Fig. 2

Rohmüll

EP 0 520 172 B1

Fig. 3

Fig. 4

Fig. 5